(19) Europäisches Patentamt
European Patent Office
Office européen des brevets

(11) Veröffentlichungsnummer: **0 054 812**
**A1**

(12) # EUROPÄISCHE PATENTANMELDUNG

(21) Anmeldenummer: **81110189.8**

(22) Anmeldetag: **05.12.81**

(51) Int. Cl.³: **C 07 D 263/10**, C 07 D 413/06, C 07 D 265/08, C 07 D 263/12, C 07 D 263/14 // A61K31/41, A61K31/535

(30) Priorität: **23.12.80 DE 3049405**

(43) Veröffentlichungstag der Anmeldung: **30.06.82**
**Patentblatt 82/26**

(84) Benannte Vertragsstaaten: **AT BE CH DE FR GB IT LI LU NL SE**

(71) Anmelder: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Vorbrüggen, Helmut, Prof., Wilkestrasse 7,**
**D-1000 Berlin 27 (DE)**
Erfinder: **Böttcher, Irmgard, Dr., Rodelbahnpfad 5,**
**D-1000 Berlin 28 (DE)**

(54) **Neue Derivate von antiphlogistisch wirksamen Carbonsäuren, ihre Herstellung und medizinische Anwendung.**

(57) Derivate antiphlogistisch wirksamer Carbonsäuren der allgemeinen Formel I

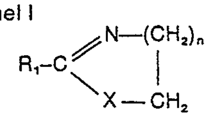

(I),

worin

$R_1$ der Rest einer antiphlogistisch wirksamen Carbonsäure $R_1COOH$,

n die Ziffern 1, 2 oder 3 und

X ein Sauerstoffatom, ein Schwefelatom oder ein gegebenenfalls alkyliertes Stickstoffatom bedeuten, sind pharmakologisch wirksame Substanzen.

**EP 0 054 812 A1**

Die Erfindung betrifft neue Derivate von antiphlogistisch wirksamen Carbonsäuren, ihre Herstellung und medizinische Anwendung.

Diese neuen Derivate von antiphlogistisch wirksamen Carbonsäuren sind gekennzeichnet durch die allgemeine Formel I

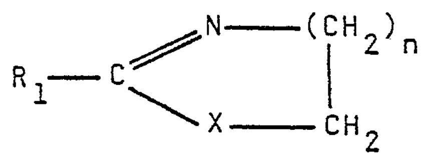

(I),

worin

$R_1$ der Rest einer antiphlogistisch wirksamen Carbonsäure $R_1COOH$,

n die Ziffern 1, 2 oder 3 und

X ein Sauerstoffatom, ein Schwefelatom oder ein gegebenenfalls alkyliertes Stickstoffatom bedeuten.

Die neuen Derivate der allgemeinen Formel I haben eine etwa gleich starke antiphlogistische Wirksamkeit wie die entsprechenden Carbonsäuren selbst, sie zeichnen sich aber dadurch aus, daß sie im Gegensatz zu den Carbonsäuren praktisch keine Magenulcera erzeugen.

Geeignete antiphlogistisch wirksame Carbonsäuren $R_1COOH$ von denen sich die neuen Derivate der allgemeinen Formel I ableiten, sind beispielsweise solche der allgemeinen Formel II a

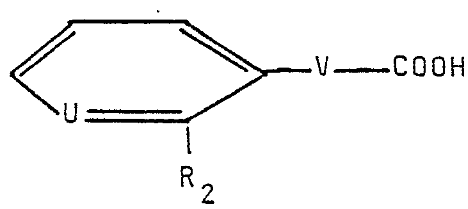

(IIa),

worin

$R_1$ eine Alkanoylgruppe mit 2 bis 6 Kohlenstoffatomen oder ein gegebenenfalls durch Halogenatome (insbesondere Chlor- oder Fluoratome), Trifluormethylgruppen und/oder Alkylgruppen mit 1 bis 4 Kohlenstoffatomen (insbesondere Methylgruppen) substituierter Phenoxyrest, Anilinorest oder 1-Naphthylaminrest darstellt und

U die Gruppierungen -CH= oder -N= und

V eine Kohlenstoff-Kohlenstoff-Bindung oder eine Methylengruppe bedeuten.

Solche Carbonsäuren der allgemeinen Formel IIa sind beispielsweise

die 2-Acetoxy-benzoesäure
die 2-[(2,6-Dichlor-phenyl)-amino]-phenylessigsäure,
die 2-[(2,4-Dichlor-1-naphthyl)-amino]-phenylessigsäure,
die 2-[(2,4-Dichlor-phenyl)-oxy]-phenylessigsäure,
die 2-[(2,3-Dimethyl-phenyl)-amino]-benzoesäure
die 2-[(3-Trifluormethyl-phenyl)-amino]-benzoesäure,
die 2-[(2,6-Dichlor-3-methyl-phenyl)-amino]-benzoesäure,
die 2-[(2-Chlor-3-methyl-phenyl)-amino]-benzoesäure,
die 2-[(3-Chlor-2-methyl-phenyl)-amino]-benzoesäure,
die 2-[(3-Trifluormethyl-phenyl)-amino]-3-pyridin-carbonsäure,
die 2-[(2-Methyl-3-trifluormethyl-phenyl)-amino]-3-pyridin-carbonsäure und
die 2-[(3-Chlor-2-methyl-phenyl)-amino]-3-pyridin-carbonsäure.

Die neuen Derivate der antiphlogistisch wirksamen Carbonsäuren der allgemeinen Formel IIa sind gekennzeichnet durch die allgemeine Formel Ia

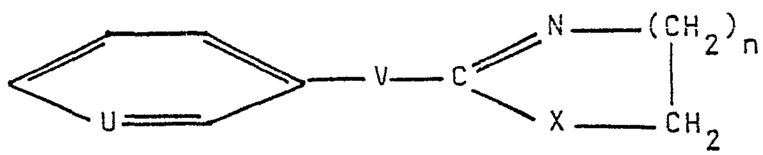

(Ia),

worin

n, $R_1$, $R_2$, U, V und X die obengenannte Bedeutung besitzen.

Geeignete antiphlogistisch wirksame Carbonsäuren $R_1COOH$ von denen sich die neuen Derivate der allgemeinen Formel I ableiten, sind ferner solche der allgemeinen Formel IIb

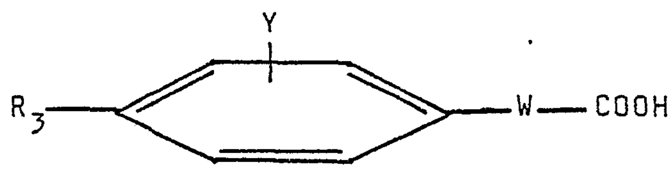

(IIb),

worin

$R_3$ eine geradkettige oder verzweigte Alkyl- oder Alkylengruppe mit 2 bis 6 Kohlenstoffatomen, eine Cycloalkylgruppe mit 3 bis 7 Kohlenstoffatomen, eine Cycloalkylalkylgruppe mit 4 bis 8 Kohlenstoffatomen, eine 2-Oxocyclopentylidenmethylgruppe, eine gegebenenfalls durch Halogenatome (vorzugsweise Fluor- oder Chloratome), Alkylgruppen mit 1 bis 4 Kohlenstoffatomen (vorzugsweise Methylgruppen) oder Trifluormethylgruppen substituierter Phenylrest, ein Alkylaminorest mit 1 bis 6 Kohlenstoffatomen, ein Alkenylrest mit 1 bis 6 Kohlenstoffatomen, ein 2,5-Dihydropyrol-1-ylrest oder ein 1-Oxo-2-isoindolinylrest darstellt und

Y ein Wasserstoffatom oder ein Halogenatom (vorzugsweise ein Fluoratom oder ein Chloratom) und

W eine Kohlenstoff-Kohlenstoff-Bindung oder eine gegebenenfalls durch eine Oxogruppe, eine Hydroxygruppe oder ein Halogenatom substituierte und/oder durch ein Sauerstoffatom oder ein Schwefelatom unterbrochene Alkylen- oder Alkylidengruppe mit 1 bis 4 Kohlenstoffatomen darstellt.

Solche Carbonsäuren sind beispielsweise:

die 4-(4-Biphenyl)-4-oxobuttersäure
die 4-(4-Biphenyl)-4-hydroxybuttersäure
die 3-(2'-Fluor-4-biphenyl)-buttersäure
die 4-(2'-Fluor-4-biphenyl)-4-oxobuttersäure
die 2-[(4'-Chlor-4-biphenyl)-methyloxy]-2-methyl-propionsäure
die 4-(4-Biphenyl)-3-hydroxybuttersäure
die 2'-Fluor-4-biphenyl-essigsäure
die 2-(2'-Fluor-4-biphenyl)-propionsäure
die 2-(2-Fluor-4-biphenyl)-propionsäure
die 2-(2-Methylpropyl)-phenyl-propionsäure
die 2-(4-Isopropyl-phenyl)-propionsäure
die 2-[4-(2-Butenyl-amino)-phenyl]-propionsäure
die 2-[4-(1-Oxo-isoindolinyl)-phenyl]-buttersäure
die 2-[4-(1-Oxo-isoindolinyl)-phenyl]-propionsäure
die 2-[4-(2,5-Dihydropyran-1-yl)-phenyl]-propionsäure
die 2-(4-Cyclohexyl-phenyl)-propionsäure
die 2-(3-Chlor-4-cyclohexyl-phenyl)-propionsäure
die 4-(3-Chlor-4-cyclohexyl-phenyl)-4-oxobuttersäure
die 2-[4-(2-Oxocyclopentyliden)-methyl-phenyl]-propionsäure oder
die 4-(2-Methylpropyl)-phenyl-essigsäure.

Die neuen Derivate der antiphlogistisch wirksamen Carbonsäuren der allgemeinen Formel IIb sind gekennzeichnet durch die allgemeine Formel I b

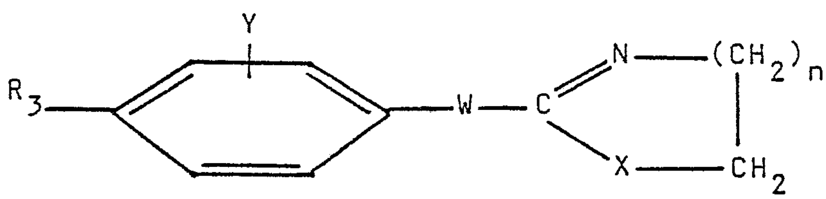

(Ib),

worin
n, $R_3$, X, Y und W die obengenannte Bedeutung besitzen.

Geeignete antiphlogistisch wirksame Carbonsäuren $R_1COOH$ von denen sich die neuen Derivate der allgemeinen Formel I weiterhin ableiten, sind solche der allgemeinen Formel IIc

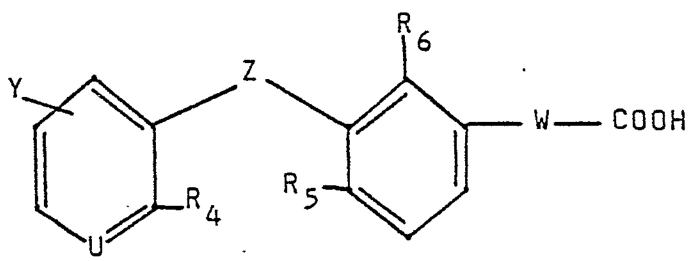

(IIc),

worin
Y, U und W die obengenannte Bedeutung besitzen und Z eine Kohlenstoff-Kohlenstoff-Bindung, eine Methylengruppe, eine Carbonylgruppe, ein Sauerstoffatom, eine Methyloxygruppe, eine Methylthiogruppe oder eine Methylaminogruppe, $R_4$ und $R_5$ zwei Wasserstoffatome oder gemeinsam ein Sauerstoffatom, ein Schwefelatom, ein Stickstoffatom, eine Aminogruppe, eine Carbonylgruppe oder eine Kohlenstoff-Kohlenstoff-Bindung darstellen, mit der Maßgabe, daß $R_4$ und $R_5$ von Z verschieden sind und worin $R_6$ ein Wasserstoffatom oder eine Aminogruppe bedeutet.

Solche Carbonsäuren der allgemeinen Formel II c sind beispielsweise

die 2-(3-Benzoylphenyl)-propionsäure
die 2-(3-Phenoxyphenyl)-propionsäure
die 2-(2-Fluorenyl)-propionsäure
die 2-(6-Chlor-2-carbazolyl)-propionsäure
die 2-(6-Oxo-3-xantenyl)-propionsäure
die 10-Methyl-2-phenothiazinyl-essigsäure
die 2-(9-Aza-3-xantenyl)-propionsäure
die 4-(3-Diphenyloxidyl)-4-oxo-buttersäure
die 2-(3-Phenoxy-2-amino-phenyl)-propionsäure
die (11-Oxo-6,11-dihydro-dibenz-[b,e]-oxepin-3-yl)-essigsäure und
die (11-Oxo-6,11-dihydro-dibenz-[b,e]-thiepin-3-yl)-essigsäure.

Die neuen Derivate der antiphlogistisch wirksamen Carbonsäuren der allgemeinen Formel II c sind gekennzeichnet durch die allgemeine Formel I c

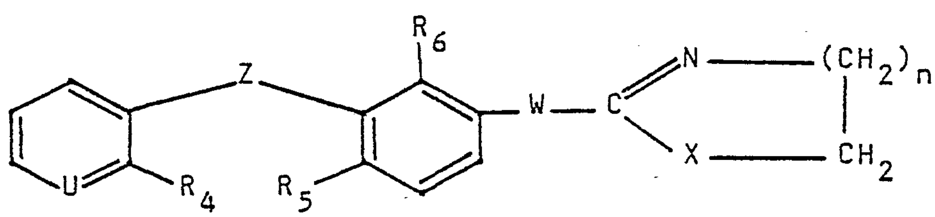

(I c),

worin
n, X, Z, U, W, $R_4$, $R_5$ und $R_6$ die obengenannte Bedeutung besitzen.

Geeignete antiphlogistisch wirksame Carbonsäuren $R_1COOH$, von denen sich die neuen Derivate der allgemeinen Formel I ableiten, sind auch solche der allgemeinen Formel II d

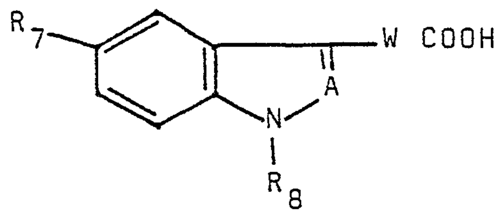

(II d),

worin

W die obengenannte Bedeutung besitzt

A ein Stickstoffatom oder eine $CH_3C$-Gruppe,

$R_7$ ein Wasserstoffatom, ein Halogenatom oder eine Methoxygruppe und

$R_8$ ein gegebenenfalls durch (vorzugsweise in para-Stellung) Halogenatome, (vorzugsweise Chloratome) Azidogruppen, Methylthiogruppen, Methylsulfinylgruppen substituierter Benzylrest, Benzoylrest oder Cinnamoylrest darstellen.

Solche Carbonsäuren sind beispielsweise

die [1-Benzyl-1H-indazol-3-yl-oxy]-essigsäure

die 1-(4-Chlorbenzoyl)-5-methoxy-2-methyl-3-indolyl-essigsäure

die 2-[1-(4-Methylthiobenzyl)-5-methoxy-2-methyl-3-indolyl-propionsäure

die 1-(4-Azido-benzoyl)-5-methoxy-2-methyl-3-indolyl-essigsäure

die 1-(4-Chlorbenzoyl)-5-methoxy-2-methyl-3-indolyl-acetoxyessigsäure und

die 1-Cinnamoyl-5-methoxy-2-methyl-3-indolyl-essigsäure.

Die neuen Derivate der antiphlogistisch wirksamen Carbonsäuren der allgemeinen Formel II d sind gekennzeichnet durch die allgemeine Formel I d

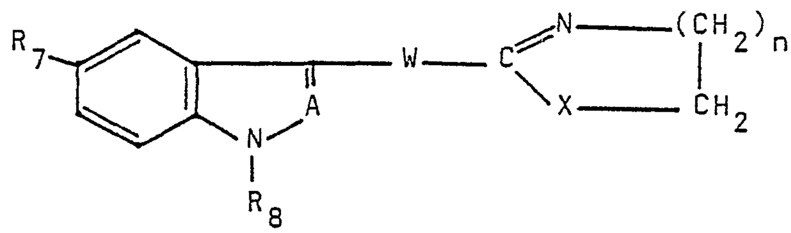

(I d)

worin
n, A, X, W, $R_7$ und $R_8$ die obengenannte Bedeutung besitzen.

Geeignete antiphlogistisch wirksame Carbonsäuren $R_1COOH$, von denen sich die neuen Derivate der allgemeinen Formel I ableiten, sind ferner solche der allgemeinen Formel II e

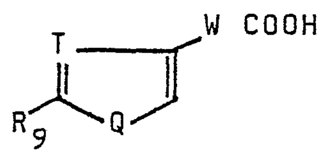

(II e),

worin
- W die obengenannte Bedeutung besitzt und
- Q ein Sauerstoffatom, ein Schwefelatom oder eine Methyliminogruppe,
- T die Gruppierungen $\geqslant N$, $\geqslant CH$ oder $\geqslant C{-}CH_3$ und
- $R_9$ eine gegebenenfalls durch ein para-ständiges Chloratom oder eine p-ständige Methylgruppe substituierter Phenylrest oder Benzoylrest bedeuten.

Solche Carbonsäuren sind beispielsweise

die 5-(4-Methylbenzoyl)-1-methyl-pyrrol-2-essigsäure
die 5-(4-Chlorbenzoyl)-1,4-dimethyl-pyrrol-2-essigsäure
und
die 3-[5-(4-Chlorphenyl)-furan-2-yl]3-hydroxy-propionsäure.

Die neuen Derivate der antiphlogistisch wirksamen Carbonsäuren der allgemeinen Formel II e sind gekennzeichnet durch die allgemeine Formel I e

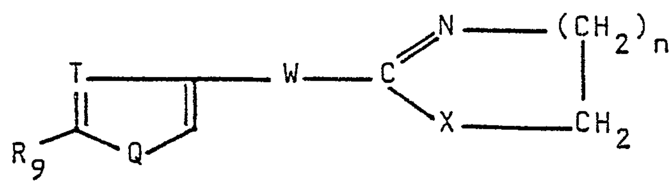

(I e),

worin
Q, T, X, W und $R_9$ die obengenannte Bedeutung besitzen.

Geeignete antiphlogistisch wirksame Carbonsäuren $R_1COOH$, von denen sich die neuen Derivate der allgemeinen Formel I ableiten, sind auch solche der allgemeinen Formel II f

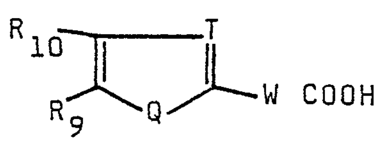

(II f),

worin
W, Q, T und $R_9$ die obengenannte Bedeutung besitzen und

$R_{10}$ ein Wasserstoffatom, eine Methylgruppe oder einen gegebenenfalls durch ein Chloratom substituierter Phenylrest darstellt.

Solche Carbonsäuren sind beispielsweise:

die 1-Methyl-5-(4-methyl-benzoyl)-pyrrol-2-essigsäure
die 5-(4-Chlorbenzoyl)-1,4-dimethyl-pyrrol-2-essigsäure
die 2-(5-Benzoyl)-2-thienyl)-propionsäure
die 3-(4,5-Dibenzyl-2-oxazolyl)-propionsäure und
die 4,5-Di-(4-chlorbenzyl)-2-oxazolyl-thioessigsäure.

Die neuen Derivate der allgemeinen Formel II f sind gekennzeichnet durch die allgemeine Formel I f

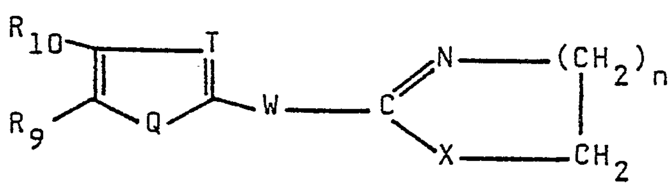

(I f),

worin
n, X, Q, T, W, $R_9$ und $R_{10}$ die obengenannte Bedeutung besitzen.

Weitere geeignete antiphlogistisch wirksame Carbonsäuren, von denen sich die neuen Derivate der allgemeinen Formel I ableiten, sind solche der allgemeinen Formel I g

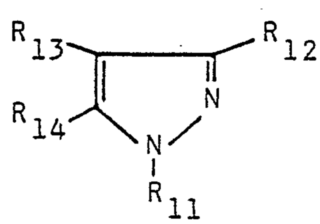

(II g),

worin

$R_1$ einen Alkylrest mit bis zu 6 Kohlenstoffatomen oder einen gegebenenfalls durch Chloratome oder Fluoratome unterbrochener Phenylrest darstellt, worin einer der Reste $R_{12}$, $R_{13}$ und $R_{14}$ einen Essigsäurerest bedeutet, ein oder zwei der Reste $R_{12}$, $R_{13}$ und $R_{14}$ einen gegebenenfalls durch Chloratome oder Fluoratome substituierter Phenylrest darstellt und gegebenenfalls einer der Reste $R_{12}$, $R_{13}$ und $R_{14}$ ein Wasserstoffatom ist.

Solche Carbonsäuren sind beispielsweise:

die 4-(4-Chlorphenyl)-1-(4-fluorphenyl)-3-pyrazolyl-essigsäure
die 1,3,5-Triphenyl-4-pyrazolyl-essigsäure
die 1,3,4-Triphenyl-5-pyrazolyl-essigsäure
die 3-(4-Chlorphenyl)-1-phenyl-4-pyrazolylessigsäure und
die 1-(2-Methyl-propyl)-3,4-diphenyl-5-pyrazolessigsäure.

Letztlich seien als geeignete antiphlogistisch wirksame Carbonsäuren $R_1COOH$, von denen sich die Derivate der allgemeinen Formel I ableiten können noch genannt:

die 3-Phenyl-benzofuran-7-yl-essigsäure
die 1-(4-Chlorphenyl)-2,5-dimethyl-pyrrol-3-essigsäure
die 4-Benzoyl-indan-1-carbonsäure
die 6-Chlor-5-cyclohexyl-indan-1-carbonsäure
die 1-Acetoxy-6-chlor-5-cyclohexyl-indan-1-carbonsäure
die 2-Isopropyl-indan-5-carbonsäure
die 4-(2-Methylpropyl)-phenyl-essigsäure
die 2-[4-(2-Methylpropyl)-phenyl)-propionsäure
die 6-Chlor-5-cyclopentylmethyl-indan-1-carbonsäure
die 2-[3-Chlor-4-(2-thenyl)-phenyl]-propionsäure

die 2-[4-(2-Thenoyl)-phenyl]-propionsäure
die 1-Chlorphenyl-2,4-dimethyl-pyrrol-3-essigsäure
die 4-(4-Chlorphenyl)-2-phenyl-5-thiazolyl-essigsäure
die 2-(6-Methoxy-2-naphthyl)-propionsäure
die 2-[2-(4-Chlorphenyl)-benzoxazol-5-yl)-propionsäure
die 4-Chlor-3-propenyloxy-phenyl-essigsäure
die 2-Hydroxy-5-(pyrrol-1-yl)-benzoesäure
die 2-Hydroxy-5-(2,4-difluorphenyl)-benzoesäure
die 5-Fluor-2-methyl-1-(4-methylsulfinyl-benzyliden)-inden-3-carbonsäure
die 2-Hydroxy-5-[2-phenyl-(benz-[f]-indol-1-yl)]-benzoesäure
die 3-(1-Butyl-dihydropyrano-[3,4-b]-indol-1-yl)-propionsäure
die 3-(1,5-Diäthyl-dihydropyrano-[3,4-b]-indol-1-yl)-propionsäure und
die 3-(1-Äthyl-dihydropyrano-[3,4-b]-indol-1-yl)-propionsäure.

Die neuen Derivate der allgemeinen Formel I können nach einem Verfahren hergestellt werden, daß dadurch gekennzeichnet ist, daß man

a) eine Carbonsäure bzw. deren Derivat der allgemeinen Formel III

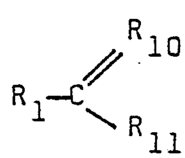

(III),

worin

$R_1$ die obengenannte Bedeutung besitzt,

$R_{10}$ ein Sauerstoffatom, ein Schwefelatom oder eine Alkyliminogruppe mit 1 bis 4 Kohlenstoffatomen im Alkylrest und

$R_{11}$ eine Hydroxygruppe, eine Alkoxygruppe mit 1 bis 6 Kohlenstoffatomen, eine Benzyloxygruppe, eine Trimethylsilyloxygruppe, eine Triäthylsilyloxygruppe oder eine Tribenzyloxygruppe bedeuten,

mit einem Amin der allgemeinen Formel IV

$$H_2N(CH_2)_nCH_2XH \qquad (IV),$$

worin

n und X die obengenannte Bedeutung besitzen kondensiert,

oder

b) eine Verbindung der allgemeinen Formel V

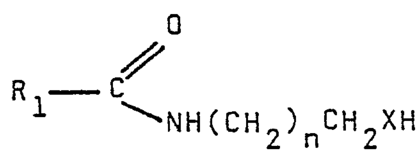

(V),

worin

$R_1$, n und X die obengenannte Bedeutung besitzen, cyclisiert.

Dieses Verfahren kann unter konventionellen Bedingungen durchgeführt werden, (Angew.Ch.88,1976,321; R.M. Acheson: An Introduction to the Chemistry of Heterocyclic Compounds; Interscience Publishers, New York, 1967, Seite 308 und 322).

Nach einer bevorzugten Methode werden die Verbindungen der allgemeinen Formel III (insbesondere Carbonsäuren selbst) in einem inerten Lösungsmittel in Gegenwart eines tertiären Amins und eines Triphenylphosphins oder Trialkylphosphins mit dem Amin der allgemeinen Formel IV umsetzt. Es bilden sich intermediär die Verbindungen der Formel V, welche im weiteren Verlauf der Reaktion zu den Verbindungen der Formel I cyclisieren.

Geeignete inerte Lösungsmittel sind beispielsweise Äther, (wie Diäthyläther, Diisopropyläther, Dioxan oder Tetrahydrofuran), dipolare aprotische Lösungsmittel (wie Acetonitril, Dimethylformamid, Hexamethylphosphorsäuretriamid, Dimethylsulfoxid oder Sulfolan), aromatische Kohlenwasserstoffe (wie Benzol, Toluol oder Xylol) chlorierte Kohlenwasserstoffe (Tetrachlormethan, Chloroform, Dichlormethan, Tetrachloräthan) und Gemische der genannten Lösungsmittel, insbesondere Acetonitril und Pyridin.

Als tertiäre Amine eignen sich vorzugsweise Trialkylamine, wie zum Beispiel Triäthylamin oder N-Heterocyclen, wie Pyridin, Lutidin oder Collidin, 2-Dimethylaminopyridin, 4-Dimethylaminopyridin, 1,5-Diazobicyclo-[4,3,0]-non-5-en 1,8-Diazobicyclo-[5,4,0]-unde-7-en oder 1,4-Diazabicyclo-[2,2,2]-octan.

Die Reaktion wird in Gegenwart von Trialkylphosphinen (Trimethylphosphin, Triäthylphosphin) oder vorzugsweise in Gegenwart von Triphenylphosphin durchgeführt.

Die erfindungsgemäßen Substanzen der allgemeinen Formel I besitzen eine gute antiinflammatorische Wirksamkeit, und zeichnen sich gegenüber den entsprechenden antiinflammatorisch wirksamen Carbonsäuren der allgemeinen Formel II dadurch aus, daß sie praktisch frei von ulcerogener Nebenwirkung sind.

Somit eignen sich die neuen Verbindungen in Kombination mit den in der galenischen Pharmazie üblichen Trägermitteln zur Behandlung zum Beispiel von Erkrankungen des rheumatischen Formenkreises (wie der Ostearthritis oder ankylosierenden Spondalitis), Asthma bronchiale, Heufieber u.a..

Bemerkenswert ist ferner, daß sich die Imidazol-Derivate der allgmeinen Formel I gemäß Anspruch 1 darüberhinaus auch zur Behandlung von Migräne und von Dysmenorhoe eignen und das Thromboserisiko mindern.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbeondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die nachfolgenden Ausführungsbeispiele dienen zur Erläuterung des erfindungsgemäßen Verfahrens.

Beispiel 1

1,237 g 2-(4-Isobutyl-phenyl)-propionsäure werden in 50 ml Acetonitril gelöst, mit 0,366 g Ethanolamin, 2,53 g Triethylamin und 3,176 g Tetrachlorkohlenstoff versetzt und dann auf 22°C bis 26°C erwärmt. Dann tropft man in die Lösung innerhalb von dreieinhalb Stunden eine Lösung von 4,72 g Triphenylphosphin in 70 ml absolutem Acetonitril zu, läßt die Reaktionsmischung 16 Stunden lang bei Raumtemperatur stehen und engt sie im Vakuum bei 30°C Badtemperatur zur Trockne ein.

Der Rückstand wird mit 100 ml Ethylether und 70 ml 2 n wässriger Natronlauge versetzt, 30 Minuten lang gerührt und die organische Phase abgetrennt. Die wässrige Phase wird noch dreimal mit je 40 ml Ethylether nachextrahiert, die vereinigten Etherphasen mit 100 ml gesättigter wässriger Kochsalzlösung gewaschen, über Natriumsulfat getrocknet und im Vakuum zur Trockne eingeengt.

Der erhaltene Rückstand wird mit 100 ml Ethylether versetzt auf dem Wasserbad erwärmt, auf Raumtemperatur gekühlt und filtriert. Das Filtrat wird eingeengt, über eine Säule aus 80 g Kieselgel mit 40 % Wasser (Firma Merck) mittels Toluol-Ethylacetat 9:1 chromatographiert und man erhält 1,18 g 2-[1-(4- Isobutyl- phenyl)-ethyl]-2-oxazolin als farbloses Öl.

$C_{15}H_{21}NO$ MG: 231,3

| | | | |
|---|---|---|---|
| Ber: | C: 77,88 | H: 9,15 | N: 6,05 |
| Gef: | C: 77,87 | H: 9,50 | N: 6,02 |

Beispiel 2

1,382 g 2-(6-Methoxy-2-naphthyl)-propionsäure (Naproxen), werden in 50 ml Acetonitril gelöst, mit 0,366 g Ethanolamin, 2,53 g Triethylamin und 3,176 g Tetrachlorkohlenstoff versetzt und dann auf 22°C bis 26°C erwärmt. Dann tropft man in die Lösung innerhalb von vier Stunden eine Lösung von 4,72 g Triphenylphosphin in 70 ml absolutem Acetonitril zu, läßt die Reaktionsmischung 3 Tage lang bei Raumtemperatur stehen und engt sie im Vakuum bei 30°C Badtemperatur zur Trockne ein.

Der Rückstand wird mit 100 ml Ethylether und 70 ml 2 n wässriger Natronlauge versetzt, 30 Minuten lang gerührt und die organische Phase abgetrennt. Die wässrige Phase wird noch dreimal mit je 40 ml Ethylether nachextrahiert, die vereinigten Etherphasen mit 100 ml gesättigter wässriger Kochsalzlösung gewaschen, über Natriumsulfat getrocknet und im Vakuum zur Trockne eingeengt.

Der erhaltene Rückstand wird mit 100 ml Ethylether versetzt auf dem Wasserbad erwärmt, auf Raumtemperatur gekühlt und filtriert. Das Filtrat wird eingeengt, über eine Säule aus 125 g Kieselgel (Firma Merck) mittels Toluol chromatographiert und man erhält 1,01 g 2-[1-(6-Methoxy-2-naphthyl)-ethyl-2-oxazolin vom Schmelzpunkt 94-96° C.

$C_{16}H_{17}NO_2$ MG: 255,3

| | | | |
|---|---|---|---|
| Ber.: | C: 75,27 | H: 6,71 | N: 5,49 |
| Gef.: | C: 74,87 | H: 6,68 | N: 5,57 |

Beispiel 3

1,15 g 2-(6-Methoxy-2-naphthyl)-propionsäure werden in 50 ml Acetonitril gelöst, mit 0,375 g 8-Amino-1-propanol, 2,02 g Triethylamin und 3,176 g Tetrachlorkohlenstoff versetzt und dann auf 22°C bis 26°C erwärmt. Dann tropft man in die Lösung innerhalb von dreieinhalb Stunden eine Lösung von 3,93 g Triphenylphosphin in 70 ml absolutem Acetonitril zu, läßt die Reaktionsmischung 72 Stunden lang bei Raumtemperatur stehen und engt sie im Vakuum bei 30°C Badtemperatur zur Trockne ein.

Der Rückstand wird mit 150 ml Ethylether und 50 ml 2 n wässriger Natronlauge versetzt, 30 Minuten lang gerührt und die organische Phase abgetrennt. Die wässrige Phase wird noch dreimal mit je 40 ml Ethylether nachextrahiert, die vereinigten Etherphasen mit 100 ml gesättigter wässriger Kochsalzlösung gewaschen, über Natriumsulfat getrocknet und im Vakuum zur Trockne eingeengt.

Der erhaltene Rückstand wird mit 50 ml Ethylether versetzt auf dem Wasserbad erwärmt, auf Raumtemperatur gekühlt und filtriert. Das Filtrat wird eingeengt, über eine Säule aus basischem Aluminiumoxid, Aktivitätsstufe IV (Firma Woelm) mittels Toluol chromatographiert und man erhält 0,745 g 2-[1-(6-Methoxy-2-naphthyl)-ethyl]-2-(5,6-dihydro-4H-1,3-oxazin) vom Schmelzpunkt 113-115° C.

$C_{17}H_{19}NO_2$ MG: 264,3

| | | | |
|---|---|---|---|
| Ber: | C: 75,81 | H: 7,11 | N: 5,2 |
| Gef: | C: 75,84 | H: 7,47 | N: 4,92 |

Beispiel 4

1,984 g 4-(4-Chlorphenyl)-1-(4-fluorphenyl)-3-pyrazolessigsäure werden in 100 ml Acetonitril gelöst, mit 0,366 g Ethanolamin, 3,04 g Triethylamin und 3,176 g Tetrachlorkohlenstoff versetzt und dann auf 22°C bis 26°C erwärmt. Dann tropft man in die Lösung innerhalb von dreieinhalb Stunden eine Lösung von 4,72 g Triphenylphosphin in 70 ml absolutem Acetonitril zu, läßt die Reaktionsmischung drei Tage lang bei Raumtemperatur stehen und engt sie im Vakuum bei 30°C Badtemperatur zur Trockne ein.

Der Rückstand wird mit 100 ml Ethylether und 70 ml 2 n wässriger Natronlauge versetzt, 30 Minuten lang gerührt und die organische Phase abgetrennt. Die wässrige Phase wird noch dreimal mit je 40 ml Ethylether nachextrahiert, die vereinigten Etherphasen mit 100 ml gesättigter wässriger Kochsalzlösung gewaschen, über Natriumsulfat getrocknet und im Vakuum zur Trockne eingeengt.

Der erhaltene Rückstand wird mit 100 ml Ethylether versetzt auf dem Wasserbad erwärmt, auf Raumtemperatur gekühlt und filtriert. Das Filtrat wird eingeengt, über eine Säule aus 80 g Kieselgel (Firma Merck) mittels Toluol chromatographiert und man erhält nach Umkristallisation aus Cyclohexan 0,65 g 2-{[4-(4-chlorphenyl)-1-(4-fluorphenyl)-3-pyrazolyl]-methyl}-2-oxazolin.

$C_{19}H_{15}ClFN_3O$ MG: 355,81

| | | | |
|---|---|---|---|
| Ber: | C: 6414 | H: 4,25 | N: 11,81 |
| Gef: | C: 64,56 | H: 4,63 | N: 11,45 |

Beispiel 5

1,59 g 2-[2,6-Dichlorphenyl)-amino]-phenylessigsaures Natrium werden in 70 ml Acetonitril gelöst, mit 0,305 g Ethanolamin, 2,02 g Triethylamin und 3,176 g Tetrachlorkohlenstoff versetzt und dann auf 22°C bis 26°C erwärmt. Dann tropft man in die Lösung innerhalb von dreieinhalb Stunden eine Lösung von 3,93 g Triphenylphosphin in 80 ml absolutem Acetonitril zu, läßt die Reaktionsmischung 22 Stunden lang bei Raumtemperatur stehen und engt sie im Vakuum bei 30°C Badtemperatur zur Trockne ein.

Der Rückstand wird mit 200 ml Toluol und 100 ml 2 n wässriger Natronlauge versetzt, 30 Minuten lang gerührt und die organische Phase abgetrennt. Die wässrige Phase wird noch dreimal mit je 100 ml Toluol nachextrahiert, die vereinigten Toluolphasen über Natriumsulfat getrocknet und im Vakuum zur Trockne eingeengt.

Der erhaltene Rückstand wird über eine Säule aus 200 g Aluminiumoxid Aktivitätsstufe IV (Firma Woelm) mittels Toluol chromatographiert und man erhält nach Umkristallisation aus Äthanol 0,655 g 2-[2-(2,6-Dichloranilino)-benzyl]-2-oxazolin vom Schmelzpunkt 141° C.

$C_{16}H_{14}N_2OCl_2$ MG: 321,22

| | | | | |
|---|---|---|---|---|
| Ber: | C: 59,83 | H: 4,39 | N: 8,72 | Cl: 22,08 |
| Gef: | C: 59,68 | H: 4,56 | N: 8,44 | Cl: 21,54 |

Beispiel 6

1,406 g 2-[(3-Trifluormethylphenyl)-amino]-benzoesäure werden in 60 ml Acetonitril gelöst, mit 0,305 g Ethanolamin, 2,02 g Triethylamin und 3,176 g Tetrachlorkohlenstoff versetzt und dann auf 22°C bis 26°C erwärmt. Dann tropft man in die Lösung innerhalb von dreieinhalb Stunden eine Lösung von 3,43 g Triphenylphosphin in 80 ml absolutem Acetonitril zu, läßt die Reaktionsmischung 20 Stunden lang bei Raumtemperatur stehen und engt sie im Vakuum bei 30°C Badtemperatur zur Trockne ein.

Der Rückstand wird mit 150 ml Ethylether und 40 ml 2 n wässriger Natronlauge versetzt, 30 Minuten lang gerührt und die organische Phase abgetrennt. Die wässrige Phase wird noch dreimal mit je 40 ml Ethylether nachextrahiert, die vereinigten Etherphasen mit 100 ml gesättigter wässriger Kochsalzlösung gewaschen, über Natriumsulfat getrocknet und im Vakuum zur Trockne eingeengt.

Der erhaltene Rückstand wird mit 100 ml Ethylether versetzt auf dem Wasserbad erwärmt, auf Raumtemperatur gekühlt und filtriert. Das Filtrat wird eingeengt, über eine Säule aus 120 g basisches Aluminiumoxid Aktivitätsstufe IV (Firma Woelm) mittels Toluol chromatographiert und man erhält 1,02 g 2-(3'-Trifluormethyl-diphenylamin)-2-oxazolin als farbloses Öl.

$C_{16}H_{13}N_2OF_3$ MG: 306,28

| | | | | |
|---|---|---|---|---|
| Ber.: | C: 62,74 | H: 4,28 | N: 9,15 | F: 18,61 |
| Gef.: | C: 63,02 | H: 4,49 | N: 8,93 | F: 18,69 |

0054812

Beispiel 7

Zu einer Lösung von 1,789 g 1-p-Chlorbenzoyl-2-methyl-5-methoxy-indol-3-essigsäure, 0,305 g Äthanolamin, 2,02 g Triäthylamin und 3,176 g Tetrachlorkohlenstoff in 100 ml absolutem Acetonitril wird bei 25° C unter Argon während dreieinhalb Stunden eine Lösung von 3,93 g Triphenylphosphin in 80 ml absolutem Acetonitril unter Rühren zugetropft. Nach 18 Stunden bei 24° C wird im Vakuum abgedampft und der kristalline Rückstand mit 200 ml Ethyläther und 75 ml 2 n wässriger Natronlauge gerührt. Nach Abtrennen der wässrigen Phase und Nachextraktion mit dreimal 40 ml Ethyläther wird die vereinigten Ätherextrakte getrocknet ($Na_2SO_4$) und eingeengt, wobei Triphenylphosphinoxyd auskristallisiert. Das Filtrat wird abgedampft und an 210 g Aluminiumoxyd-Aktivität IV (basisch: Firma Woelm) mit Toluol chromatographiert, wobei nach 300 ml Vorlauf die nächsten 800 ml Eluat 1,38 g hellgelbes öliges (4-Chlorphenyl)-[5-methoxy-2-methyl-3(oxazolin-2-yl-methyl)-1-indolyl]-keton ergeben.

$C_{21}H_{19}N_2O_3Cl$

| | | | |
|---|---|---|---|
| Ber.: | C: 65,88 | H: 5,00 | N: 7,32 |
| Gef.: | C: 65,51 | H: 4,82 | N: 7,45 |

## Patentansprüche

1. Derivate antiphlogistisch wirksamer Carbonsäuren der allgemeinen Formel I

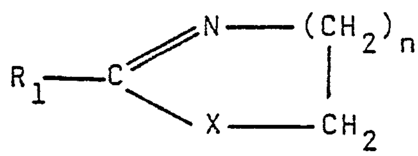 (I),

worin

$R_1$ der Rest einer antiphlogistisch wirksamen Carbonsäure $R_1COOH$,

n die Ziffern 1, 2 oder 3 und

X ein Sauerstoffatom, ein Schwefelatom oder ein gegebenenfalls alkyliertes Stickstoffatom bedeuten.

2. Derivate antiphlogistisch wirksamer Carbonsäuren der allgemeinen Formel I gemäß Anspruch 1, dadurch gekennzeichnet, daß X ein Sauerstoffatom bedeutet.

3. Derivate antiphlogistisch wirksamer Carbonsäuren der allgemeinen Formel I gemäß Anspruch 1 und 2, dadurch gekennzeichnet, daß $R_1$ einen 1-(4-Isobutylphenyl)-ethylrest, einen 1-(6-Methoxy-2-naphthyl)-ethyl-rest, einen 4-(4-chlorphenyl)-1-(4-fluorphenyl)-3- pyrazolyl-rest, einen 2-(2,6-Dichloranilino)-benzyl-rest, einen 2-(3-Trifluormethyl-anilino)-phenyl-rest, einen 1-(4-Chlorbenzoyl)-5-methoxy-2-methyl-indolyl-methylrest darstellt.

4. 1-(4-Isobutyl-phenyl)-ethyl-2-oxazolin

5. 1-(6-Methoxy-2-naphthyl)-ethyl-2-oxazolin

6. 1-(6-Methoxy-2-naphthyl)-ethyl-2-(5,6-dihydro-4H-1,3-oxazin).

7. [4-(4-chlorphenyl)-1-(4-fluorphenyl)-3-pyrazolyl]-methyl-2-oxazolin.

8. [2-(2,6-Dichloranilino)-benzyl]-2-oxazolin.

9. 2-(3-Trifluormethyl-anilino)-phenyl-2-oxazolin.

10. (4-Chlorphenyl)-[5-methoxy-2-methyl-3-(oxazolin-2-ylmethyl)-1-indolyl]-keton.

11. Verfahren zur Herstellung von Derivaten antiphlogistisch wirksamer Carbonsäuren der allgemeinen Formel I

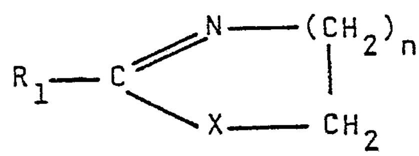

(I),

worin

$R_1$ der Rest einer antiphlogistisch wirksamen Carbonsäure $R_1COOH$,

n die Ziffern 1, 2 oder 3 und

X ein Sauerstoffatom, ein Schwefelatom oder ein gegebenenfalls alkyliertes Stickstoffatom bedeuten,

dadurch gekennzeichnet, daß man

a) eine Carbonsäure bzw. deren Derivat der allgemeinen Formel III

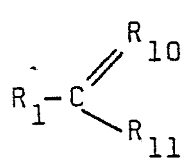

(III),

worin

$R_1$ die obengenannte Bedeutung besitzt,

$R_{10}$ ein Sauerstoffatom, ein Schwefelatom oder eine Alkyliminogruppe mit 1 bis 4 Kohlenstoffatomen im Alkylrest und

$R_{11}$ eine Hydroxygruppe, eine Alkoxygruppe mit 1 bis 6 Kohlenstoffatomen, eine Benzyloxygruppe, eine Trimethylsilyloxygruppe, eine Triäthylsilyloxygruppe oder eine Tribenzyloxygruppe bedeuten,

mit einem Amin der allgemeinen Formel IV

$$H_2N(CH_2)_nCH_2XH \qquad (IV),$$

worin

n und X die obengenannte Bedeutung besitzen kondensiert, oder

b) eine Verbindung der allgemeinen Formel V

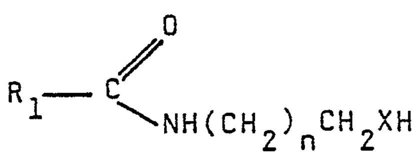 (V),

worin

$R_1$, n und X die obengenannte Bedeutung besitzen, cyclisiert.

Europäisches Patentamt

# EUROPÄISCHER RECHERCHENBERICHT

0054812
Nummer der Anmeldung
EP 81 11 0189.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 2 151 799 (A.H. ROBINS CO.) | |
| | * Seite 4, Zeilen 1 bis 5; Formel I * | 1,2 |
| | * Seite 4, Zeile 20 bis Seite 5, Zeile 6 * | 11 |
| | -- | |
| X | DE - B2 - 1 617 618 (MERCK PATENT GMBH) | |
| | * Spalte 4, Zeilen 1 bis 7; Formel 1; Beispiele * | 1 |
| | * Spalte 2, Zeilen 21 bis 31 * | 11 |
| | -- | |
| A | Patent Abstracts of Japan<br>Band 1, Nr. 27, 28. März 1977<br>Seite 1295C76<br>& JP - A - 51 - 141835 | 8 |
| | -- | |
| A | Patent Abstracts of Japan<br>Band 1, Nr. 61, 15. Juni 1977<br>Seite 774C77<br>& JP - A - 52 - 23058 | |
| | ---- | |

**KLASSIFIKATION DER ANMELDUNG (Int. Cl.)**

C 07 D 263/10
C 07 D 413/06
C 07 D 265/08
C 07 D 263/12
C 07 D 263/14
// A 61 K 31/41
A 61 K 31/535

**RECHERCHIERTE SACHGEBIETE (Int. Cl.)**

A 61 K 31/405
A 61 K 31/41
A 61 K 31/42
C 07 D 263/08
C 07 D 263/10
C 07 D 263/12
C 07 D 263/14
C 07 D 265/08
C 07 D 277/08
C 07 D 277/10

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 10-03-1982 | PHILLIPS |